# EUROPEAN PATENT SPECIFICATION

(11) **EP 2 640 413 B1**
(45) Date of publication and mention of the grant of the patent: **12.06.2019**
(21) Application number: 11784653.5
(22) Date of filing: 16.11.2011
(51) Int. Cl.: A61K 38/48, C07K 14/745

(54) **A PROCESS FOR REDUCTION AND/OR REMOVAL OF FXI AND FXIA FROM SOLUTIONS CONTAINING SAID COAGULATION FACTORS**
VERFAHREN ZUR VERRINGERUNG UND/ODER ENTFERNUNG VON FXI UND FXIA AUS LÖSUNGEN MIT BESAGTEN GERINNUNGSFAKTOREN
PROCÉDÉ DE RÉDUCTION ET/OU D'EXTRACTION DE FXI ET DE FXIA DE SOLUTIONS CONTENANT LESDITS FACTEURS DE COAGULATION

(30) Priority: 16.11.2010 EP 10191398
(43) Date of publication of application: 25.09.2013
(73) Proprietor: Octapharma AG, 8853 Lachen (CH)
(72) Inventor: SCHULTZ, Petra, A-1100 Vienna (AT); GRUBER, Gerhard, A-1100 Vienna (AT); BAL, Frederic, F-67381 Lingolsheim (FR); MARKS, Frank, 31832 Springe (DE); WINGE, Stefan, S-11275 Stockholm (SE)
(74) Representative: Patent- und Rechtsanwälte Ullrich & Naumann
(86) International application number: PCT/EP2011/070257
(87) International publication number: WO 2012/066036

(56) References cited:
- WO-A1-2006/128497
- WO-A1-2008/081025
- TAKEHIKO KOIDE ET AL: "Isolation and characterization of bovine factor XI (plasma thromboplastin antecedent)", BIOCHEMISTRY, vol. 16, no. 10, 1 May 1977 (1977-05-01), pages 2279-2286, XP55018316, ISSN: 0006-2960, DOI: 10.1021/bi00629a037
- TAIT J F ET AL: "PRIMARY STRUCTURE REQUIREMENTS FOR THE BINDING OF HUMAN HIGH MOLECULAR WEIGHT KININOGEN TO PLASMA PREKALLIKREIN AND FACTOR XI", JOURNAL OF BIOLOGICAL CHEMISTRY, vol. 262, no. 24, 1987, pages 11651-11656, XP002627224, ISSN: 0021-9258
- BOUMA B N ET AL: "HUMAN BLOOD COAGULATION FACTOR-XI PURIFICATION PROPERTIES AND MECHANISM OF ACTIVATION BY ACTIVATED FACTOR-XII", JOURNAL OF BIOLOGICAL CHEMISTRY, vol. 252, no. 18, 1977, pages 6432-6437, XP002627223, ISSN: 0021-9258
- BURNOUF-RADOSEVICH M ET AL: "A THERAPEUTIC, HIGHLY PURIFIED FACTOR XI CONCENTRATE FROM HUMAN PLASMA", TRANSFUSION, AMERICAN ASSOCIATION OF BLOOD BANKS, BETHESDA, MD, US, vol. 32, no. 9, 1 January 1992 (1992-01-01), pages 861-867, XP009084439, ISSN: 0041-1132, DOI: DOI:10.1046/J.1537-2995.1992.32993110761.X
- MASHIKO H ET AL: "PURIFICATION OF FACTOR XI AND SOME PROPERTIES OF ACTIVATED FACTOR XI FROM PORCINE PLASMA", AGENTS AND ACTIONS SUPPLEMENTS, BIRKHAEUSER VERLAG, BASEL, CH, vol. 38, no. PART 2, 1 January 1992 (1992-01-01), pages 249-256, XP009084254, ISSN: 0379-0363
- SAITO H ET AL: "PARTIAL PURIFICATION OF PLASMA THROMBOPLASTIN ANTECEDENT FACTOR-XI AND ITS ACTIVATION BY TRYPSIN", JOURNAL OF CLINICAL INVESTIGATION, vol. 52, no. 4, 1973, pages 850-861, XP002627225, ISSN: 0021-9738
- BURNOUF T ET AL: "Affinity chromatography in the industrial purification of plasma proteins for therapeutic use", JOURNAL OF BIOCHEMICAL AND BIOPHYSICAL METHODS, AMSTERDAM, NL, vol. 49, no. 1-3, 30 October 2001 (2001-10-30), pages 575-586, XP002353092, ISSN: 0165-022X, DOI: DOI:10.1016/S0165-022X(01)00221-4
- FUJIKAWA K: "Historical perspective of factor XI", THROMBOSIS RESEARCH, TARRYTOWN, NY, US, vol. 115, no. 6, 1 January 2005 (2005-01-01), pages 441-450, XP004803852, ISSN: 0049-3848, DOI: DOI:10.1016/J.THROMRES.2004.10.013
- Teschner W et al: "Reduction of Procoagulant Activities in the Gammagard Liquid/KIOVIG Process", Baxter Innovations GmbH , 15 September 2002 (2002-09-15), Retrieved from the Internet: URL:http://www.bo-conf.com/ppb13/present/p apers/103.pdf
- A S Wolberg ET AL: "Coagulation factor XI is a contaminant in intravenous immunoglobulin preparations", American journal of hematology, 1 September 2000 (2000-09-01), pages 30-34, XP055193310, UNITED STATES DOI: 10.1002/1096-8652(200009)65:1<30::AID-AJH5 >3.0.CO;2-J Retrieved from the Internet: URL:http://www.ncbi.nlm.nih.gov/pubmed/109 36860

## Description

The present invention pertains to a process for reduction and/or removal of FXI and FXIa from solutions containing said coagulation factors.

Coagulation factor XI (FXI) is well known to be a protein involved in the coagulation of blood and represents one part of the intrinsic pathway of the coagulation cascade. FXI is the precursor of activated FXI (FXIa), which is the active compound during coagulation. Therefore it is essential to remove FXIa from pharmaceutical preparations being intravenously applied to patients as said FXIa may unintentionally start coagulation leading to life endangering thrombotic events. A concentrate of FXI and/or FXIa might on the other hand be beneficial for patients suffering from a disease related to lack or insufficient activity of FXI or for patients experiencing heavy blood loss where fast and effective coagulation is vitally important. Such concentrate can also be beneficial for patients suffering from inhibitory antibodies to coagulation factors, such as in Haemophilia A and B. Those inhibitors can cause bleeding events, thus patients require agents to support coagulation and wound closure.

It is thus an objective of the present invention to remove or at least reduce FXI and/or FXIa from solutions, which contain FXI and/or FXIa.

Several methods to prepare a FXI concentrate are known. Bouma and Griffin published in THE JOURNAL OF BIOLOGICAL CHEMISTRY, 1977, vol. 252, no. 18, pp. 6432-6437 a process for purification of FXI from human plasma by five chromatographic steps. The chromatographic materials used in a chronological order are DEAE-Sephadex®, QAE-Sephadex®, SP-Sephadex® (twice) and finally Concanavalin A bound to Sepharose® All buffers used contain Polybrene® and benzamidine.

M. BURNOUF-RADOSEVICH AND T. BURNOUF reported in TRANSFUSION, 1992, 32, pp. 861-867 the production of a FXI lyophilisate by filtration of cryo-poor plasma over a negatively charged filter (Zeta plus 50S), which adsorbs FXI, elution of the adsorbed FXI and chromatography of the eluate on a cation exchange resin (Sulfate-Sepharose® Fast Flow). Eluted FXI was formulated with antithrombin and heparin prior to lyophilisation.

Hiroshl Mashiko and Hidenobu Takahashi disclosed in BIOL. CHEM. HOPPE-SEYLER, 1994, vol. 375, pp. 481-484 a production method for porcine FXI and FXIa based on high molecular mass kininogen-affinity chromatography and chromatography on Q-Sepharose®. To avoid contact activation and overcome proteolytical digestion they added Polybrene® and benzamidine to the buffers used. They also reported that they failed to purify FXI by Heparin-Sepharose®-affinity chromatography.

Another paper of Hiroshl Mashiko and Hidenobu Takahashi discussed in AGENTS AND ACTIONS SUPPLEMENTS,(BIRKHAEUSER VERLAG, BASEL, CH), 1992, vol. 38, part 2, pp. 249-256 a production method for porcine FXI and FXIa based on chromatography with high molecular mass kininogen, Q-Sepharose® and Protein A-Superose® with Polybrene® and benzamidine present in buffers.

Tait and Fujikawa disclosed in JOURNAL OF BIOLOGICAL CHEMISTRY, 1981, vol. 262, no. 24, pp. 11651-11656 a method to purify FXI and prekallikrein from human plasma by three chromatographic steps and the use of Polybrene® and benzamidine in the buffers. A synthetic peptide representing one part of the light chain of high molecular weight kininogen was immobilized on a carrier and used to isolate prekallikrein, FXI and some carryover of IgG from plasma. Prekallikrein and FXI were subsequently separated by heparin-agarose chromatography and finally polished by CM-Sephadex® to obtain apparently pure fractions of FXI and prekallikrein.

Saito et al. presented in THE JOURNAL OF CLINICAL INVESTIGATION, vol. 52, pp. 850-861, 1973 a purification method for FXI consisting of adsorption of plasma on Ca₃(PO₄)₂, multiple ammonium sulfate fractionation, and successive chromatography on QAE-Sephadex® (twice), Sephadex®-G150, and SP-Sephadex® with Polyprene® present to prevent activation of FXI.

Koide et al. published a process relating to the isolation of FXI from blood or blood plasma in order to provide a FXI concentrate in BIOCHEMISTRY, vol. 16, no. 10, pp. 2279 to 2286, 1977. During the disclosed process FXI is removed from a source solution using several chromatographic separations and numerous wash steps with large volumes of wash buffers comprising Polybrene® and benzamindine.

### Summary of the invention

The present invention provides a process for reducing the content of FXI and FXIa from a solution containing said proteins and as main component immunoglobulins. This is achieved by adsorption of said proteins on adsorbing material suitable for affinity chromatography. Said materials suitable for affinity chromatography are composed of polysaccarides, e.g. dextrane, heparin or heparan, linked to matrix material (e.g. zeolithes or polymers such as acrlyates and saccarides), in particular a gel used for heparin affinity chromatography such as e.g. Heparin Sepharose (™) FF or Toyopearl AF Heparin 650 M(™).

The solution containing source may be any liquid containing FXI and FXIa derived from blood or blood plasma or liquids derived from biotechnological processes. Known but not limiting examples of such solutions are: cryo-poor plasma, intermediates of the Cohn process (e.g. reconstituted paste I+II+III) and its derivatives, intermediates of the Kistler-Nitschmann process (e.g. reconstituted precipitate A) and its derivatives, or solutions resulting from recombinant protein expression but also solutions which are primarily composed of other proteins, wherein FXI and FXIa represent impurities, which may be the case for solutions of immunoglobulin-gamma (IgG).

The process for reduction and/or removal of FXI and FXIa from a source solution containing said coagulation factors and as main component immunoglobulins comprises the following steps:
a) applying the source solution to an affinity chromatographic matrix in a loading buffer of 10-18 mS conductivity, wherein heparin or heparan is linked to the matrix material;
b) allowing adsorption of FXI and FXIa to the chromatographic matrix in a buffer of 10-18 mS conductivity;
c) separating the liquid deprived of FXI and FXIa from the chromatographic matrix, and
d) optionally, eluting FXI and FXIa from the chromatographic matrix with an elution buffer of low to intermediate ionic strength containing 0.2 to 1.4 NaCl to provide an FXI and/or FXIa eluate,
wherein the process is performed in the absence of Polybrene® (hexadimethrine bromide) and benzamidine, and
wherein the liquid deprived of FXI and FXIa separated in step c) is characterized by an FXI content of 0.00 to 0.05 IU FXI/ml and an FXIa content of less than 5 mU FXIa/ml.

It is also possible to modify step a) in so far that the active sites of the matrix or gel are either already saturated or are allowed to saturate with antithrombin during loading of the matrix or gel with FXI and FXIa.

Adsorption may either be performed as batch adsorption wherein the source solution is mixed with the adsorbent, stirred and FXI and FXIa loaded onto the adsorbent are removed from the supernatant by known processes like sedimentation, filtration or centrifugation. An alternative procedure is packing of adsorption material into a chromatographic column and application of the source solution to load the adsorbent with FXI and FXIa.

In an embodiment of the invention, silicates selected from the group of silica, perlites, zeolithes or diatomaceous earth may be additionally used as adsorbens of FXI and FXIa.

In a further embodiment, an additional adsorption medium selected from the group of aluminium hydroxide, aluminium oxide hydroxide or aluminium oxide may be used.

It is also possible to combine an adsorption preformed in batch modus for instance with diatomaceous earth or aluminum hydroxide as adsorption material with an adsorption performed in a chromatographic column with Heparin Sepharose(™) as adsorbent.

In still another embodiment of the invention, one adsorption on heparin or heparan linked to a matrix material is performed after the chromatographic material was preconditioned with Antithrombin-III.

Loaded adsorbent is carefully washed with a washing buffer to avoid desorption of FXI and/or FXIa and resulting wash solution may be added to the flow-through and/or a supernatant to be further processed and to optimize recovery of other compounds of interest, such as immunoglobulins, in particular IgG, in this FXI/FXIa-depleted solution. A FXI/FXIa-depleted solution processed over Heparin Sepharose(™) typically contains from 0.00 to 0.05 IU FXI/ml. The content of FXIa expressed in international units (IU) of such a depleted solution is typically less than 5 mU FXIa/ml, even more particular from 0.0 to 1.0 mU FXIa/ml.

Further processing of the FXI/FXIa-depleted solution may incorporate one or more virus inactivation steps, examples given are solvent/detergent treatment (S/D treatment), UV-radiation, pasteurization, low pH incubation, caprylate precipitation or nanofiltration. Other steps include chromatographic steps, concentration to obtain a concentrate of a pharmaceutically active compound, formulation and filling, which are known from manufacturing of various proteins such as immunoglobulins, in particular IgG, albumin, fibrinogen, antithrombin or alpha-1-antitrypsin, and are mandatory in order to obtain pharmaceutical compositions and depend on the product to be produced.

FXI and FXIa may be eluted from the loaded adsorbent, in particular if the adsorbent is an affinity chromatography gel with heparin or heparan attached to the matrix. Elution of FXI and FXIa from an affinity chromatography gel is performed with an elution buffer consisting of 0.2-1.4 M NaCl, in particular 0.25-1.0 M NaCl, even more particular 0.25-0.5 M NaCl and 0.003-0.03 M phosphate or equivalent ion strength. Thus, afforded solution containing FXI and FXIa can also be virus inactivated by methods mentioned above and concentrated by ultra/diafiltration to obtain a concentrate containing FXI and FXIa. Said concentrate may further be formulated with adjuvants to obtain a pharmaceutical composition capable of treating diseases related to lack or inactivity of FXI or FXIa.

It was surprising to find FXI and FXIa levels of the effluent of a heparin chromatography gel below the detection limit of said proteins although antithrombin was allowed to break through. It was expected that FXI and FXIa would have been replaced by antithrombin, in particular the antithrombin-β isoform, as it is known to strongly bind to heparin and heparan affinity gels. It was even more astonishing that it was possible to selectively elute FXI and FXIa from the antithrombin saturated heparin affinity gel with elution buffers of low to intermediate ionic strength, essentially containing 0.2-1.4 M NaCl, while it is necessary to elute antithrombin-III (AT-III) with buffers of higher ionic strength, essentially containing more than 1.5 M NaCl, in particular at about 2.0 M NaCl. This feature allows either separate elution of FXI/FXIa followed by elution of AT-III or co-elution of a mixture containing FXI, FXIa and AT-III when eluting with a buffer of sufficiently high ionic strength to also elute AT-III.

It was even more surprising to find unnecessary the addition of Polybrene® and benzamidine to buffers, as generally taught by prior art literature to avoid contact activation and overcome proteolytical digestion.

One objective of the present invention is performed to the best advantage by preconditioning affinity chromatographic gels with dextrane, heparin or heparan linked to the matrix material with antithrombin-III (AT-III). Preconditioning may be performed to such an extent that the active sites of the chromatographic material are saturated with AT-III. This procedure is especially beneficial as the binding capacity of the affinity gel for FXI and FXIa is improved and binding of other coagulation factors is prohibited or at least hindered to a high degree. It is thus possible to remove FXI and FXIa selectively from accompanying proteins and obtain a solution of FXI and FXIa devoid of other coagulation factors after elution from the affinity gel.

A concentrate of a pharmaceutically active component obtainable by the process of the invention as well as a pharmaceutical composition obtained thereof is also disclosed herein.

In the concentrate of the pharmaceutically active component, the pharmaceutically active component is IgG.

### Detailed description of the invention

The present invention provides a process for reducing the content of FXI and FXIa from a solution containing said proteins and as main component immunoglobulins. This is achieved by adsorption of said proteins on adsorbing material selected materials suitable for affinity chromatography, in particular a gel used for heparin- or heparan-affinity chromatography such as e.g. Heparin Sepharose (™) FF or Toyopearl AF Heparin 650 M(™).

The solution containing source may be any liquid containing FXI and FXIa derived from blood or blood plasma or liquids derived from biotechnological processes. Known but not limiting examples of such solutions are cryo-poor plasma, intermediates of the Cohn process (e.g. reconstituted paste I+II+III) and its derivatives, intermediates of the Kistler-Nitschmann process (e.g. reconstituted precipitate A) and its derivatives or solutions resulting from recombinant protein expression but also solutions which are primarily composed of other proteins, wherein FXI and FXIa represent impurities, which may be the case for solutions of immunoglobulin-gamma (IgG).

The process for reduction and/or removal of FXI and FXIa from solutions containing said coagulation factors and as main component immunoglobulins comprises the following steps:
a) applying the source solution to an affinity chromatographic matrix in a loading buffer of 10-18 mS conductivity, wherein heparin or heparan is linked to the matrix material;
b) allowing adsorption of FXI and FXIa to the chromatographic matrix in a buffer of 10-18 mS conductivity;
c) separating the liquid deprived of FXI and FXIa from the chromatographic matrix, and
d) optionally, eluting FXI and FXIa from the chromatographic matrix with an elution buffer of low to intermediate ionic strength containing 0.2 to 1.4 NaCl to provide an FXI and/or FXIa eluate,
wherein the process is performed in the absence of Polybrene® (hexadimethrine bromide) and benzamidine and
wherein the liquid deprived of FXI and FXIa separated in step c) is characterized by an FXI content of 0.00 to 0.05 IU FXI/ml and an FXIa content of less than 5 mU FXIa/ml.

It is also possible to modify step a) in so far that the active sites of the matrix or gel are either already saturated or are allowed to saturate with antithrombin during loading of the matrix or gel with FXI and FXIa.

An alternative procedure is packing of adsorption material into a chromatographic column and application of the source solution to load the adsorbent with FXI and FXIa.

In an embodiment of the invention, diatomaceous earth may be additionally used as adsorbens of FXI and FXIa.

Steps a) and b) are performed by loading the proteins in a buffer with a conductivity of 10-18 mS, in particular with a conductivity of 14-17 mS, onto the chromatographic resin (Heparin-Sepharose ™ FF). Loaded adsorbent is carefully washed with a washing buffer of the same conductivity to avoid desorption of FXI and/or FXIa and resulting wash solution may be added to the flow-through and/or a supernatant to be further processed and to optimize recovery of IgG present in the flow-through as FXI/FXIa-depleted solution. The FXI/FXIa-depleted solution processed over Heparin Sepharose(™) typically contains from 0.00 to 0.05 IU FXI/ml. The content of FXIa expressed in international units (IU) of such a depleted solution is typically less than 5 mU FXIa/ml, even more particular from 0.0 to 1.0 mU FXIa/ml.

Further processing of the FXI/FXIa-depleted solution may incorporate one or more virus inactivation steps, examples given are solvent/detergent treatment (S/D treatment), as disclosed in EP-A-131 740, UV-radiation, pasteurization, low pH incubation, caprylate precipitation or nanofiltration. Other steps include chromatographic steps, concentration to obtain a concentrate of a pharmaceutically active compound, formulation and filling, which are known from manufacturing of various proteins such as immunoglobulins, in particular IgG, albumin, fibrinogen, antithrombin or alpha-1-antitrypsin, and are mandatory in order to obtain pharmaceutical compositions and depend on the product to be produced.

FXI and FXIa may be eluted from the loaded adsorbent with an elution buffer consisting of 0.36 M NaCl and 0.01 M phosphate. Thus afforded solution containing FXI and FXIa can also be virus inactivated by methods mentioned above and concentrated by ultra/diafiltration to obtain a concentrate containing FXI and FXIa. Said concentrate may further be formulated with adjuvants to obtain a pharmaceutical composition capable of treating diseases related to lack or inactivity of FXI or FXIa.

### Examples

### Factor XIa activity assay

A recombinant coagulation factor IX (void of FIXa) is activated to FIXa by FXIa present in the sample. In the presence of phospholipids and calcium ions FIXa forms an enzyme complex with thrombin-activated FVIII:C which is in excess in the assay solution. This enzyme complex subsequently activates FX, which is also present in the assay solution, to Factor Xa (FXa). The generated amount of FXa is measurable by commercially available substrates and directly proportional to the FXIa concentration in the sample. Quantification is done by comparison with a calibration curve.

It has to be mentioned that this assay also indicates the activities of FIXa and FXa when samples from early stages of the plasma fractionation process, such as cryo-poor plasma, are measured. It is thus comprehensible that the summarized activities of FXIa, FIXa and FXa are indicated for such samples, with the prerequisite that FIXa and FXa are present in the sample.

### Example 1:

Starting material was processed over Heparin Sepharose FF packed in a column to allow adsorption of FXI and FXIa on the chromatographic material. The IgG containing flow-through was brought in contact with Hyflo, i.e. diatomaceous earth, centrifuged to remove loaded Hyflo and the IgG containing supernatant was subsequently processed to the intermediate paste I+II+III. Reconstitution of thus produced intermediate revealed 0.02 IU FXI/ml and less than 1 mU FXIa/ml.

### Example 2:

Paste I+II+III was produced in the same way as Example 1 with the exemption of omitting the FXI/FXIa-capture on diatomaceous earth. Determination of FXI and FXIa revealed a content of 0.05 IU FXI/ml and 3.5 mU FXIa/ml.

Tables 1-3 represent analytical results of samples before and after chromatography wherein runs 2-5 were performed with a reduced load of starting material for the heparin gel compared to run 1.

**Table 1: Analysis of starting material.**

| **Sample A** - **Starting material** | | **Reduced column load** | | | |
|---|---|---|---|---|---|
| | **run 1** | **run 2** | **run 3** | **run 4** | **run 5** |
| IqG [g/L] | 7.12 | 6.94 | 6.65 | 5.47 | 7.02 |
| Factor XI [IU/mL] | 1.03 | 1.00 | 1.00 | 0.94 | 0.92 |
| Factor XIa [mU/mL] | 2.5 | 1.6 | 1.6 | 1.9 | 1.6 |

**Table 2: Sample analysis of several experiments according to Examples 1 (run 1, run 4 and run 5) and 2 (run 2 and run 3).**

| **Sample B - after Heparin Sepharose Chrom.** | | **Reduced column load** | | | |
|---|---|---|---|---|---|
| | **run 1** | **run 2** | **run 3** | **run 4** | **run 5** |
| IgG [g/L] | 6.42 | 6.5 | 6.38 | 5.66 | 6.95 |
| Factor XI [IU/mL] | 0.05 | < 0.01 | < 0.01 | < 0.01 | < 0.01 |
| Factor XIa [mU/mL] | 3.5 | <1.0 | <1.0 | <1.0 | <1.0 |

**Table 3: Sample analysis of several experiments according to Example 1**

| **Sample C - after Heparin Sepharose and Hyflo treatment** | | **Reduced column load** | | | |
|---|---|---|---|---|---|
| | **run 1** | **run 2** | **run 3** | **run 4** | **run 5** |
| IqG [g/L] | 4.79 | - | - | 4.98 | 4.43 |
| Factor XI [IU/mL] | 0.02 | - | - | < 0.01 | < 0.01 |
| Factor XIa [mU/mL] | <1.0 | - | - | <1.0 | <1.0 |

## Claims

1. A process for reduction and/or removal of FXI and FXIa from a source solution containing said coagulation factors and as main components immunoglobulins, comprising the following steps:
a) applying the source solution to an affinity chromatographic matrix in a loading buffer of 10-18 mS conductivity, wherein heparin or heparan is linked to the matrix material;
b) allowing adsorption of FXI and FXIa to the chromatographic matrix in a buffer of 10-18 mS conductivity;
c) separating the liquid deprived of FXI and FXIa from the chromatographic matrix, and
d) optionally, eluting FXI and FXIa from the chromatographic matrix with an elution buffer of low to intermediate ionic strength containing 0.2 to 1.4 NaCl to provide an FXI and/or FXIa eluate,
wherein the process is performed in the absence of Polybrene® (hexadimethrine bromide) and benzamidine and
wherein the liquid deprived of FXI and FXIa separated in step c) is **characterized by** an FXI content of 0.00 to 0.05 IU FXI/ml and an FXIa content of less than 5 mU FXIa/ml.

2. The process according to claim 1 wherein the active sites of the affinity chromatography gel are either already saturated or are allowed to saturate with antithrombin.

3. The process according to claim 1 or claim 2, wherein the process comprises an adsorption performed in batch modus with diatomaceous earth as adsorption material.

4. The process according to claim 1 or claim 2, wherein an additional adsorption medium selected from the group of aluminium hydroxide, aluminium oxide hydroxide or aluminium oxide is used.

5. The process according to any one of claims 1 to 4, wherein one adsorption on heparin or heparan linked to matrix material is performed after the matrix material is preconditioned with Antithrombin-III.

6. The process according to any one of claims 1 to 5, wherein the source solution primarily contains immunoglobulin-γ.

7. The process according to any one of claims 1 to 6, wherein the liquid deprived of FXI and FXIa is further subjected to at least one virus inactivation step selected from solvent/detergent treatment, UV-radiation, pasteurization, low pH incubation, caprylate precipitation or nanofiltration to obtain a virus inactivated solution deprived of FXI and FXIa.

8. The process according to any one of claims 1 to 7 , wherein the, optionally virus inactivated, liquid deprived of FXI and FXIa is concentrated to obtain a concentrate of a pharmaceutically active component, which is optionally formulated to obtain a pharmaceutical composition.

9. The process according to any one of claims 6 to 8, wherein
(i) following step c) the matrix material is washed with a buffer of 10-18 mS conductivity and the flow-through of step (a) and the effluent of the washing procedure is combined with the liquid deprived of FXI and FXIa and further processed with at least one virus inactivation step employing tri-N-butyl phospate and a detergent;
(ii) followed by a concentrating step; and
(iii) optionally, formulation of the obtained immunoglobulin-γ containing concentrate.

10. The process according to any one of claims 1 to 9, wherein FXI and FXIa are eluted from the chromatographic matrix with an elution buffer consisting of 0.36 M NaCl and 0.01 M phosphate.

11. The process according to any one of claims 1 to 10, wherein the FXI and FXIa eluate is subjected to a virus inactivation step.

12. The process according to any one of claims 1 to 11, wherein the FXI and FXIa eluate is concentrated, in particular by ultra/diafiltration, to obtain a concentrate containing FXI and FXIa.

## Patentansprüche

1. Ein Verfahren zur Verringerung und/oder Entfernung von FXI und FXIa aus einer Ausgangslösung, die die genannten Koagulationsfaktoren und als Hauptkomponenten Immunglobuline enthält, umfassend die folgenden Schritte:
a) Aufbringen der Ausgangslösung auf eine affinitätschromatographische Matrix in einem Ladepuffer mit einer Leitfähigkeit von 10-18 mS, wobei Heparin oder Heparan an das Matrixmaterial gebunden ist;
b) Zulassen der Adsorption von FXI und FXIa an die chromatographische Matrix in einem Puffer mit einer Leitfähigkeit von 10 - 18 mS;
c) Trennen der Flüssigkeit, der FXI und FXIa entzogenen wurde, von der chromatographischen Matrix; und
d) gegebenenfalls Eluieren von FXI und FXIa von der chromatographischen Matrix mit einem Elutionspuffer mit geringer bis mittlerer lonenstärke, der 0,2 bis 1,4 M NaCl enthält, um ein FXI- und/oder FXIa-Eluat bereitzustellen,
wobei das Verfahren in Abwesenheit von Polybrene® (Hexadimethrinbromid) und Benzamidin durchgeführt wird, und
wobei die in Schritt c) getrennte Flüssigkeit, der FXI und FXIa entzogenen wurde, durch einen FXI-Gehalt von 0,00 bis 0,05 IU FXI/ml und einen FXIa-Gehalt von weniger als 5 mU FXIa/ml gekennzeichnet ist.

2. Das Verfahren nach Anspruch 1, wobei die aktiven Zentren des Affinitätschromatographie-Gels entweder bereits, mit Antithrombin, gesättigt sind oder gesättigt werden können.

3. Das Verfahren nach Anspruch 1 oder Anspruch 2, wobei das Verfahren eine Adsorption umfasst, die im Batch-Modus mit Kieselgur als Adsorptionsmaterial durchgeführt wird.

4. Das Verfahren nach Anspruch 1 oder Anspruch 2, wobei ein zusätzliches Adsorptionsmedium verwendet wird, das aus der Gruppe Aluminiumhydroxid, Aluminiumoxidhydroxid oder Aluminiumoxid ausgewählt ist.

5. Das Verfahren nach einem der Ansprüche 1 bis 4, wobei eine Adsorption an Heparin oder Heparan, das an Matrixmaterial gebunden ist, durchgeführt wird, nachdem das Matrixmaterial mit Antithrombin-III vorkonditioniert wurde.

6. Das Verfahren nach einem der Ansprüche 1 bis 5, wobei die Ausgangslösung hauptsächlich Immunglobulin-γ enthält.

7. Das Verfahren nach einem der Ansprüche 1 bis 6, wobei die Flüssigkeit, der FXI und FXIa entzogen wurde, ferner mindestens einem Virusinaktivierungsschritt unterzogen wird, ausgewählt aus Lösungsmittel-/Detergensbehandlung, UV-Strahlung, Pasteurisierung, Inkubation bei niedrigem pH-Wert, Caprylatfällung oder Nanofiltration, um eine virusinaktivierte Lösung zu erhalten, der FXI und FXIa entzogen wurde.

8. Das Verfahren nach einem der Ansprüche 1 bis 7, wobei die gegebenenfalls virusinaktivierte Flüssigkeit, der FXI und FXIa entzogen wurde, konzentriert wird, um ein Konzentrat einer pharmazeutisch aktiven Komponente zu erhalten, die gegebenenfalls formuliert wird, um eine pharmazeutische Zusammensetzung zu erhalten.

9. Das Verfahren nach einem der Ansprüche 6 bis 8, wobei
(i) nach Schritt c) das Matrixmaterial mit einem Puffer mit einer Leitfähigkeit von 10-18 mS gewaschen wird und der Durchfluss von Schritt (a) und der Ausfluss des Waschvorgangs mit der Flüssigkeit, der FXI und FXIa entzogen wurde, vereinigt und mit mindestens einem Virusinaktivierungsschritt unter Verwendung von Tri-n-butylphosphat und einem Detergens weiterverarbeitet wird;
(ii) gefolgt von einem Konzentrierungsschritt; und
(iii) gegebenenfalls Formulierung des erhaltenen Immunglobulin-γ-haltigen Konzentrats.

10. Das Verfahren nach einem der Ansprüche 1 bis 9, wobei FXI und FXIa mit einem Elutionspuffer bestehend aus 0,36 M NaCl und 0,01 M Phosphat von der chromatographischen Matrix eluiert werden.

11. Das Verfahren nach einem der Ansprüche 1 bis 10, wobei das FXI und FXIa-Eluat einem Virusinaktivierungsschritt unterzogen wird.

12. Das Verfahren nach einem der Ansprüche 1 bis 11, wobei das FXI und FXIa-Eluat insbesondere durch Ultra/Diafiltration konzentriert wird, um ein Konzentrat zu erhalten, das FXI und FXIa enthält.

## Revendications

1. Procédé de réduction et/ou d'élimination du FXI et FXIa à partir d'une solution source contenant lesdits facteurs de la coagulation et, en tant que composants principaux, des immunoglobulines, comprenant les étapes suivantes consistant à :
a) appliquer la solution source à une matrice de chromatographie d'affinité dans un tampon de chargement ayant une conductivité de 10-18 mS, où de l'héparine ou de l'héparane est lié(e) au matériau de matrice ;
b) permettre l'adsorption du FXI et FXIa sur la matrice de chromatographie dans un tampon ayant une conductivité de 10-18 mS ;
c) séparer le liquide dépourvu de FXI et FXIa à partir de la matrice de chromatographie, et
d) facultativement, éluer le FXI et FXIa à partir de la matrice de chromatographie avec un tampon d'élution ayant une force ionique faible à intermédiaire contenant du NaCl 0,2 à 1,4 afin d'obtenir un éluat de FXI et/ou FXIa
où le procédé est réalisé en l'absence de Polybrene® (bromure d'hexadiméthrine) et de benzamidine et
où le liquide dépourvu de FXI et FXIa séparé à l'étape c) est **caractérisé par** une teneur en FXI de 0,00 à 0,05 IU de FXI/ml et une teneur en FXIa inférieure à 5 mU de FXIa/ml.

2. Procédé selon la revendication 1, dans lequel les sites actifs du gel de chromatographie d'affinité sont déjà saturés ou sont soumis à une saturation par l'antithrombine.

3. Procédé selon la revendication 1 ou la revendication 2, où le procédé comprend une adsorption réalisée en mode batch avec de la terre de diatomée en tant que matériau d'adsorption.

4. Procédé selon la revendication 1 ou la revendication 2, dans lequel un milieu d'adsorption supplémentaire sélectionné dans le groupe de l'hydroxyde d'aluminium, l'hydroxyde d'oxyde d'aluminium ou l'oxyde d'aluminium est utilisé.

5. Procédé selon l'une quelconque des revendications 1 à 4, dans lequel une adsorption sur de l'héparine ou de l'héparane lié(e) à un matériau de matrice est réalisée après un pré-conditionnement du matériau de matrice avec l'antithrombine-III.

6. Procédé selon l'une quelconque des revendications 1 à 5, dans lequel la solution source contient principalement l'immunoglobuline-γ.

7. Procédé selon l'une quelconque des revendications 1 à 6, dans lequel le liquide dépourvu de FXI et FXIa est en outre soumis à au moins une étape d'inactivation de virus sélectionnée parmi un traitement par un solvant/détergent, une irradiation UV, une pasteurisation, une incubation à faible pH, une précipitation au caprylate ou une nanofiltration afin d'obtenir une solution inactivée en virus dépourvue de FXI et FXIa.

8. Procédé selon l'une quelconque des revendications 1 à 7, dans lequel le liquide dépourvu de FXI et FXIa, facultativement inactivé en virus, est concentré pour obtenir un concentré d'un composant pharmaceutiquement actif, qui est facultativement formulé afin d'obtenir une composition pharmaceutique.

9. Procédé selon l'une quelconque des revendications 6 à 8, dans lequel
(i) après l'étape c) , le matériau de matrice est lavé avec un tampon ayant une conductivité de 10-18 mS et l'écoulement de l'étape (a) et l'effluent de la procédure de lavage est combiné avec le liquide dépourvu de FXI et FXIa et en outre traité avec au moins une étape d'inactivation de virus employant du phosphate de tri-N-butyle et un détergent ;
(ii) suivi d'une étape de concentration ; et
(iii) facultativement, la formulation du concentré contenant l'immunoglobuline-γ obtenu.

10. Procédé selon l'une quelconque des revendications 1 à 9, dans lequel le FXI et le FXIa sont élués de la matrice de chromatographie avec un tampon d'élution consistant en du NaCl 0,36 M et du phosphate 0,01 M.

11. Procédé selon l'une quelconque des revendications 1 à 10, dans lequel l'éluat de FXI et FXIa est soumis à une étape d'inactivation de virus.

12. Procédé selon l'une quelconque des revendications 1 à 11, dans lequel l'éluat de FXI et FXIa est concentré, en particulier par une ultra/diafiltration, afin d'obtenir un concentré contenant le FXI et FXIa.
